# EUROPÄISCHE PATENTSCHRIFT

(11) **EP 1 717 305 B1**
(45) Veröffentlichungstag und Bekanntmachung des Hinweises auf die Patenterteilung: **26.09.2007**
(21) Anmeldenummer: 05008977.0
(22) Anmeldetag: 25.04.2005
(51) Int. Cl.: C12M 1/107, B01F 7/00

(54) **Biogasanlagen-Serviceschacht für einen Fermenterbehälter einer Biogasanlage**
Manhole for a fermenter in a biogas facility
Élement de regard pour le fermenteur d'une installation de biogaz

(43) Veröffentlichungstag der Anmeldung: 02.11.2006
(73) Patentinhaber: U.T.S. Umwelt-Technik-Süd GmbH, 84419 Obertaufkirchen (DE)
(72) Erfinder: Bürger, Adam, 84405 Grüntegernbach (DE)
(74) Vertreter: Neubauer, Hans-Jürgen

(56) Entgegenhaltungen:
- DE-A1- 19 951 959
- DE-C1- 19 714 342
- DE-U1- 20 015 808

## Beschreibung

Die Erfindung betrifft einen Biogasanlagen-Serviceschacht für einen Fermenterbehälter einer Biogasanlage nach dem Oberbegriff des Anspruchs 1.

Um an Blogasanlagen Reparatur- und/oder Wartungsarbeiten an einer Rühreinheit eines Rührwerks ohne Beeinträchtigung mittels Biogasen mit hoher Arbeitssicherheit auf einfache Weise auch bei einem fortlaufenden Fermentationsprozess durchführen zu können, ist aus der gattungsbildenden DE 197 14 342 C1 bereits ein Biogasanlagen-Serviceschacht für einen Fermenterbehälter einer Biogasanlage bekannt. Dort ist der Serviceschacht als gasdichte Abdeckung domartig über einer in einer Fermenterbehälterabdeckung ausgebildeten Serviceöffnung angebracht, durch die hindurch eine Rühreinheit aus dem Fermenterbehälter heraus in den durch wenigstens eine gasdicht verschließbare Zugangsöffnung zugänglichen Serviceschacht-Innenraum bewegbar ist. Die Rühreinheit ist an einer im Fermenterbehälter vertikal gehaltenen und mit einem oberen Stützstangenende in einen Serviceschacht-Innenraum geführte Stützstange höhenverstellbar geführt und gehalten.

Konkret weist der Fermenterbehälter hier eine durch eine Betondecke gebildete, horizontal ausgerichtete Fermenterbehälter-Deckenwand auf, auf der der Biogasanlagen-Serviceschacht mit einem Abstand von einem Randkantenbereich der Serviceöffnung aufgesetzt ist, um im Innenraum des Serviceschachtes um die Serviceöffriung herum eine Auflagekante auszubilden. Bei in den Innenraum des Serviceschachtes gebrachter Rühreinheit kann dann die Serviceöffnung von der geöffneten Zugangsöffnung her mit auf den Auflagekanten aufliegenden Überlagen, wie z. B. Brettern, abgedeckt werden, auf die dann eine Gummimatte aufgelegt werden kann, die eine Aussparung entsprechend dem Stützstangenquerschnitt sowie einen von dieser Aussparung bis zu einem Rand der Gummimatte durchgehenden Schlitz aufweist. Dadurch kann der Fermenterinnenraum für Reparatur- und/oder Montagearbeiten weitgehend gasdicht von dem Serviceschacht-Innenraum begehbar abgetrennt werden. Ohne derartige Abdeckungen, d. h. im normalen Betriebszustand, bei dem keine Wartungs- und/oder Reparaturarbeiten durchgeführt werden, befindet sich somit auch Biogas im Serviceschacht-Innenraum, so dass eine gasdichte Anbindung des Serviceschachtes an der horizontalen Fermenterbehälter-Deckenwand erforderlich ist.

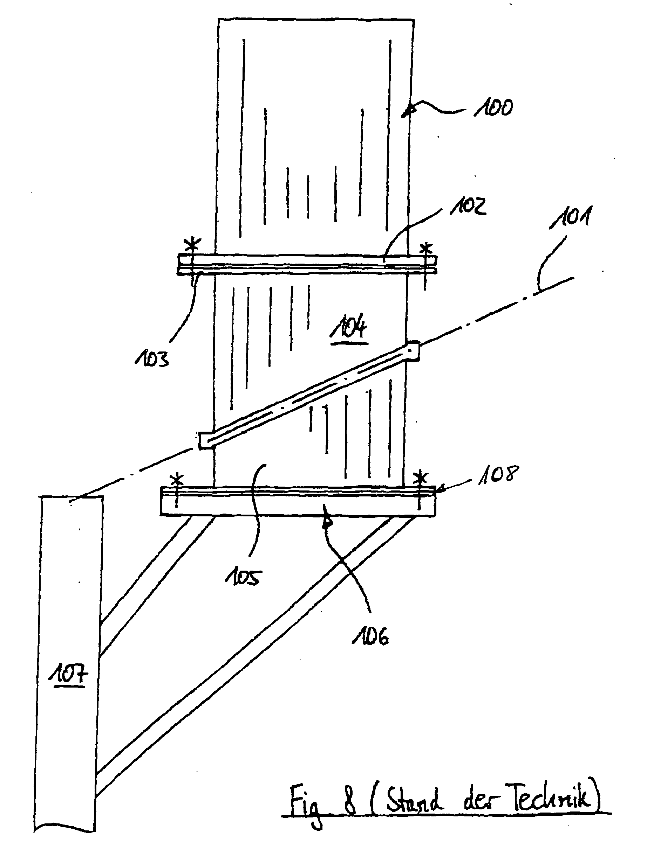

Neben diesen massiven Fermenterbehälter-Betondeckenwänden ist es ferner bekannt, zur Fermenterbehälterabdeckung sogenannte Foliendächer zu verwenden, die fermenterbehälteröffnungsrandseitig festgelegt sind und sich dann unter einem vorgegebenen Neigungswinkel zu einer zentral über der Fermenterbehältermitte angeordnetem höchsten Dachspitze hin verlaufend erstrecken. Derartige Foliendächer ermöglichen die Speicherung einer größeren Gasmenge und sind zudem innerhalb gewisser vorgebbarer Bereiche flexibel, wobei das Foliendachmaterial so stabil ausgebildet ist, dass ein derartiges Foliendach jederzeit begehbar ist und auch extremen Witterungsverhältnissen standhält. Übliche Dachneigungen derartiger Foliendächer liegen z. B. zwischen 15 bis 30 Grad. Um in Verbindung mit derartigen gegen die Horizontale geneigten Foliendächern analog zu der zuvor in Verbindung mit horizontalen Fermenterbehälter-Dockenwänden geschilderten Art und Weise Reparatur- und/oder Wartungsarbeiten an der Rühreinheit vornehmen zu können, ist es, wie dies in der Fig. 8 schematisch und beispielhaft dargestellt ist, bereits allgemein bekannt, den zuvor beschriebenen Biogasanlagen-Serviceschacht 100 oberhalb des Foliendaches 101 mittels eines am unteren Serviceschachtende umlaufenden Flanschbereiches 102 mit einem Gegen-Flanschbereich 103 ines Rohrstückes 104 gasdicht zu verbinden. Dieses Rohrstück 104 ist mit einem dem Serviceschacht abgewandten Rohrende 105 unter Ausbildung der Serviceöffnung durch das Foliendach 101 geführt und dort an einer Tragkonsole 106 festgelegt und abgestützt, die ihrerseits wiederum an einer Fermenterbehälter-Seitenwand 107 festgelegt ist. Um dieses einen Adapter ausbildende Rohrstück gasdicht durch das Foliendach hindurchzuführen wird der die Serviceöffnung umgrenzende Folienrandbereich zwischen zwei Klemmleisten 108 verklemmt, von denen eine gasdicht mit der Rohraußenwand verschweißt ist. Das Dokument DE 20015808 U beschreibt einen ähnlichen Serviceschacht für einen Fermenterbehälter mit einem Foliendach.

Aufgabe der Erfindung ist es daher, einen alternativen Biogasanlagen-Serviceschacht für einen Fermenterbehälter einer Biogasanlage mit einem gegen die Horizontale geneigten Foliendach zur Verfügung zu stellen, der einfacher und kostengünstiger aufgebaut ist und der im Hinblick auf Installations- und/oder Wartungs- und/oder Reparaturarbeiten an einer Rühreinheit eine erhöhte und verbesserte Funktionalität aufweist.

Diese Aufgabe wird gelöst mit den Merkmalen des Anspruchs 1.

Nach Anspruch 1 ist die Fermenterbehälterabdeckung durch ein gegen die Horizontale geneigtes Fermenterbehälter-Foliendach gebildet, durch das der Serviceschacht mit einem unteren Schachtende unter Ausbildung der Serviceöffnung gasdicht hindurch und in den Fermenterbehäiterinnenraum geführt sowie dort festgelegt ist.

Ein derartiger erfindungsgemäßer Biogasanlagen-Serviceschacht für ein gegen die Horizontale geneigtes Fermenterbehälterfoliendach kann auf einfache Weise abgedichtet werden, da der Serviceschacht hier erst unterhalb des Foliendaches und damit im Fermenterbehälterinnenraum an z. B. einer Tragkonsole angeschlossen wird, so dass an diesem Anschlussbereich des Serviceschachtes keine gasabdichtenden Maßnahmen erforderlich sind und lediglich eine Gasdichtheit zwischen dem die Serviceöffnung umgebenden Folienrandbereich und dem Serviceschacht hergestellt werden braucht.

Durch die erfindungsgemäße Verlagerung der Serviceschacht-Anschlussebene in den Fermenterbehälterinnenraum wird zudem eine äußerst foliendachnahe Anordnung der gasdicht verschließbaren Zugangsöffnung erzielt, was wiederum zur Folge hat, dass im Vergleich zum sehr hoch bauenden Aufbau gemäß dem Stand der Technik nach Fig. 8 größere Rühreinheiten bzw. Antriebsmotoren mit größeren Rührflügeldurchmessem über den Serviceschacht in den Fermenterbehälterinnenraum auch bei niedrig bauendem Aufbau ein- bzw. ausgebracht werden können.

Ein weiterer wesentlicher Vorteil eines derartigen erfindungsgemäß niedrig bauenden Serviceschachtes ist zudem, dass durch die in den Fermenterbehälterinnenraum verlagerte Serviceschacht-Anschlussebene insbesondere in Verbindung mit einer Serviceschacht-Tragstruktur weniger Blechverkleidung erforderlich ist bzw. das als Rohrstück ausgebildete Adapterstück eingespart werden kann.

Durch diesen insgesamt niedrig bauenden Aufbau werden gleichzeitig insbesondere in Verbindung mit relativ groß bauenden und einen großen Behälterdurchmesser aufweisenden Fermenterbehältern günstige Kraftverhältnisse erzielt, da aufgrund der dann kürzeren Stützstange weniger Schwingungen in den Bereich des Serviceschachtes eingeleitet werden bzw. kürzere Hebelwege vorhanden sind.

Insgesamt gesehen kann daher ein derartiger Biogasanlagen-Serviceschacht für gegen die Horizontale geneigte Fermenterbehälter Foliendächer wesentlich einfacher und preisgünstiger hergestellt werden als dies bisher der Fall war, wobei zudem auch noch die Funktionalität hinsichtlich der Wartungs- und/oder Reparatur- und/oder Installationsarbeiten wesentlich erhöht worden ist.

Durch die mittels der Tragkonsole nach Anspruch 2 zur Verfügung gestellte horizontale Anschlussebene ist zudem sichergestellt, dass ein vom Grundaufbau her identischer Biogasanlagen-Serviceschacht sowohl in Verbindung mit gegen die Horizontale geneigten Foliendächern als auch in Verbindung mit horizontalen Fermenterbehälter-Deckenwänden verwendet werden kann. Durch dieses modulare System kann die Bauteilvielfalt vorteilhaft reduziert werden.

Nach Anspruch 3 ist vorgesehen, dass das Foliendach mit seinem die Serviceöffnung umgebenden Folienrandbereich gasdicht zwischen mehreren, vorzugsweise zwei dem Serviceschacht zugeordneten und um diesen umlaufenden Klemmleisten verklemmt ist. Dadurch wird in dem einzigen abzudichtenden Bereich eine besonders funktionssichere Gasdichtheit geschaffen.

Gemäß einer weiteren besonders bevorzugten konkreten Ausgestaltung nach Anspruch 4 ist vorgesehen, dass der Serviceschacht eine rahmen- und/oder gestellartige Serviceschacht-Tragstruktur aufweist, die wenigstens in dem das Foliendach nach außen überragenden Bereich mit einer Außenhaut beplankt bzw. ummantelt ist. Die Außenhaut ist hierbei z. B. durch ein Edelstahlblech gebildet, das korrosionsbeständig und zudem auch beständig gegen die aggressiven Bedingungen im Fermenterbehälter-Innenraum selbst ist. Die Außenhaut bzw. Außenbleche sind, insbesondere aus Dichtigkeitsgründen, bevorzugt mit der Serviceschacht-Tragstruktur verschweißt, wobei die Tragstruktur selbst, ebenso wie die Tragkonsole, bevorzugt als Schweißkonstruktion durch Träger, insbesondere Hohlprofilträger mit vorzugsweise rechteckigem bzw. quadratischem Querschnitt ausgebildet sind. Die Serviceschacht-Tragstruktur selbst kann ebenso wie die Tragkonsole aus einem Edelstahl oder aber auch Kunststoff hergestellt sein.

Eine vorteilhafte Funktionsintegration ergibt sich mit den Merkmalen des Anspruchs 5, nach denen eine im montierten Zustand des Serviceschachtes untere Klemmleiste der beiden Klemmleisten integraler Bestandteil der Außenhaut ist. Dies kann beispielsweise durch Umbördeln eines unteren Außenhaut-Randkantenbereichs erfolgen. In diesem Fall braucht dann nur noch eine obere Klemmleiste auf die untere Klemmleiste aufgesetzt und mit dieser unter gasdichter Verklemmung des die Serviceöffnung begrenzenden Folienrandbereiches verbunden zu werden. Grundsätzlich bestünde hier auch die Möglichkeit, die obere Klemmleiste integral mit der Außenhaut auszubüden, was jedoch dann eine Montage der zweiten Klemmleiste von der Foliendachunterseite her erforderlich machen würde, was zwar relativ aufwendig ist, als äquivalente Ausführungsform aber ausdrücklich auch vom Schutzumfang des Anspruchs 5 mit umfasst sein soll.

Gemäß einer weiteren besonders bevorzugten Ausgestaltung bildet diese in die Außenhaut integrierte Klemmleiste dann den unteren Abschluss der Außenhautbeplankung, so dass der Serviceschacht lediglich noch mit seinem im montierten Zustand die Serviceöffnung nach unten hin überragenden unteren Tragstrukturende unter das Foliendach und in den Fermenterbehälterinnenraum geführt ist.

Grundsätzlich kann der Serviceschacht jede erdenkliche Form aufweisen, z. B. einen runden oder eckigen Grundriss aufweisen oder zylinderförmig bzw. kastenförmig aufgebaut sein. Die Abmessungen des Serviceschachtes sind ebenfalls je nach Anwendungsfall beliebig wählbar, so z. B. mannshoch bzw. auch nur hüfthoch ausführbar. Nach Anspruch 6 weist der Serviceschacht bevorzugt einen einfach herzustellenden rechteckförmigen Grundriss mit einem in jedem Eckbereich angeordneten Vertikalträger als Bestandteil der Serviceschacht-Tragstruktur auf, Diese Vertikalträger sind am im montierten Zustand des Serviceschachtes oberen Endbereich der Serviceschacht-Tragstruktur miteinander durch horizontale Querträger zu einem rechteckförmigen und die Tragstruktur insgesamt stabilisierenden Tragstruktur-Rahmenbereich verbunden. Grundsätzlich kann dieser Rahmenbereich auch beabstandet vom oberen freien Tragstrukturende ausgebildet sein, wobei jedoch die endseitige Anordnung eine insgesamt vorteilhaftere Beplankung der Tragstruktur mit der Außenhaut erlaubt. Weiter können die Vertikalträger am im montierten Zustand des Serviceschachtes unteren Endbereich der Serviceschacht-Tragstruktur horizontale Flanschplatten zur Festlegung an der die horizontale Anschlussebene aufweisenden Tragkonsole aufweisen. Über derartige horizontale Flanschplatten wird eine sehr einfache und zuverlässige Anbindung des Serviceschachtes an der die horizontale Anschlussebene aufweisenden Tragkonsole möglich.

Ein besonders steifer Aufbau ergibt sich mit den Merkmalen des Anspruchs 7, in dem zwischen den jeweils benachbarten Vertikalträgern der Tragstruktur des Serviceschachtes zudem gegebenenfalls weitere Versteifungsstreben angeordnet werden.

Besonders bevorzugt ist eine Ausführungsform nach Anspruch 8, bei der die Tragkonsole zur Ausbildung der horizontalen Anschlussebene einen dem Serviceschacht unmittelbar zugeordneten und im montierten Zustand einen rechteckförmigen Grundriss aufweisenden, horizontalen, insbesondere durch einzelne Querträger gebildeten Konsolenrahmen aufweist, auf dem die Serviceschacht-Tragstruktur mittels der an den Vertikafträgern angeordneten horizontalen Flanschplatten festgelegt werden kann. Die Festlegung erfolgt hierbei insbesondere durch eine Verschraubung. Insgesamt wird damit eine besonders stabile und schnell und einfach sowie funktionssichere Anbindung des Serviceschachtes an der Tragstruktur mit bevorzugter horizontaler Anschlussebene zur Verfügung gestellt.

Nach Anspruch 9 kann die Tragkonsole vier von den Eckbereichen des im montierten Zustand in einer Horizontalebene liegenden Konsolenrahmens ausgehende Diagonalstreben aufweisen, die zur Freigabe der Serviceöffnung vom Fermenterbehälter-Innenraum ausgehend jeweils parallel zueinander verlaufen und mit ihren freien Enden an einer Seitenwand des Fermenterbehälters festgelegt sind. Durch die zueinander parallele Ausrichtung dieser Diagonalstreben kann zudem einfach sichergestellt werden, dass der Bereich unterhalb der Serviceöffnung freigehalten wird, so dass die Rühreinheit entlang der Stützstange ungehindert nach oben und nach unten verstellt werden kann. Auch in Verbindung mit einer derartigen, vorzugsweise als Schweißkonstruktion ausgebildeten Tragkonsole können wiederum Querverstrebungen zwischen jeweils benachbarten Streben vorgesehen sein, um die Tragkonsole noch stabiler und steifer auszubilden. In diesem Zusammenhang kann nach Anspruch 10 auch vorgesehen sein, dass die Tragkonsole im montierten Zustand zusätzlich auf einander in Fermenterbehälterumfangsrichtung gesehen gegenüberliegenden Konsolenseiten mit in der Draufsicht schräg verlaufenden Versteifungsstreben versteift ist, die einerseits an der Tragkonsole und andererseits an der Fermenterbehälter-Seitenwand angebunden sind.

Für eine großflächige Serviceschacht-Zugangsöffnung ist nach Anspruch 11 vorgesehen, dass diese in dem aufgrund der Foliendachneigung die größte Fläche aufweisenden Serviceschachtbereich oberhalb des Foliendachs ausgebildet ist. Dabei handelt es sich stets um den zum Fermenterbehälterrandbereich hin weisenden Serviceschachtbereich. Bevorzugt ist die Zugangsöffnung durch eine gasdicht verschließbare Zugangsklappe oder Zugangstür gebildet.

Um möglichst große Rühreinheiten in den Bereich des Serviceschachtes heben zu können, ist nach Anspruch 12 vorgesehen, dass die Stützstange in einem außermittigen Randbereich durch die Serviceöffnung hindurchgeführt ist. Das obere Stützstangenende kann an einem Wandbereich des Serviceschachtes verdrehbar gehalten und abgestützt sein, um ein Verschwenken der Rühreinheit in einer Horizontalebene des Fermenterbehälters zu ermöglichen. Zur Verdrehung der Stützstange ist nach Anspruch 13 vorgesehen, dass die Stützstange von außerhalb des Serviceschachtes mittels einer gasdicht durch die Serviceschachtwand geführten und mittels dem oberen Stützstangenende gekoppelten bzw. Bestandteil desselben bildenden Verdreheinrichtung verdrehbar ist.

Nach Anspruch 14 ist zur Höhenverstellung der einen Antrieb und einen Rührer aufweisenden Rühreinheit eine Verstelleinrichtung vorgesehen, die bevorzugt durch eine mittels einer Kurbel betätigbaren Seilzuganordnung gebildet ist. Diese Verstelleinrichtung ist an der Stützstange angeordnet und im Serviceschacht-Innenraum aufgenommen.

In Verbindung mit dem Serviceschacht können zudem noch weitere Funktionsteile vorgesehen sein, so z. B. ein bullaugenartiges Kontrollglas, das einen Blick in das Fermenterbehälterinnere erlaubt bzw. auch eine Gasfackel, um einen kritischen Gasüberdruck im Fermenterbehälter durch Abfackeln des Biogases abzubauen.

Die Erfindung wird nachfolgend anhand einer Zeichnung näher erläutert.

Es zeigen:
- Fig. 1: schematisch einen erfindungsgemäßen Biogasanlagen-Serviceschacht, der an einer Tragkonsole vormontiert ist,
- Fig. 2: den Zusammenbau nach Fig. 1 kurz vor der Montage an einer Fermenterbehälter-Seitenwand,
- Fig. 3: den Zusammenbau nach Fig. 1 und 2 im montierten Zustand mit montierter Stützstange,
- Fig. 4: einen erfindungsgemäßen Biogasanlagen-Serviceschacht im fertig montierten Zustand mit zwischen Klemmleisten gasdicht zu verklemmenden Foliendachbereichen,
- Fig. 5: schematisch eine perspektivische Ansicht eines erfindungsgemäßen Biogasanlagen-Serviceschachtes (ohne Verschlusstür) mit integral an dem Außenblech ausgebildeter erster Klemmleiste,
- Fig. 6: schematisch eine perspektivische Darstellung einer Tragkonsole,
- Fig. 7: schematisch eine Draufsicht auf den Zusammenbau gemäß Fig. 3, und
- Fig. 8: schematisch eine Biogasanlagen-Serviceschachtkonstruktion gemäß dem Stand der Technik.

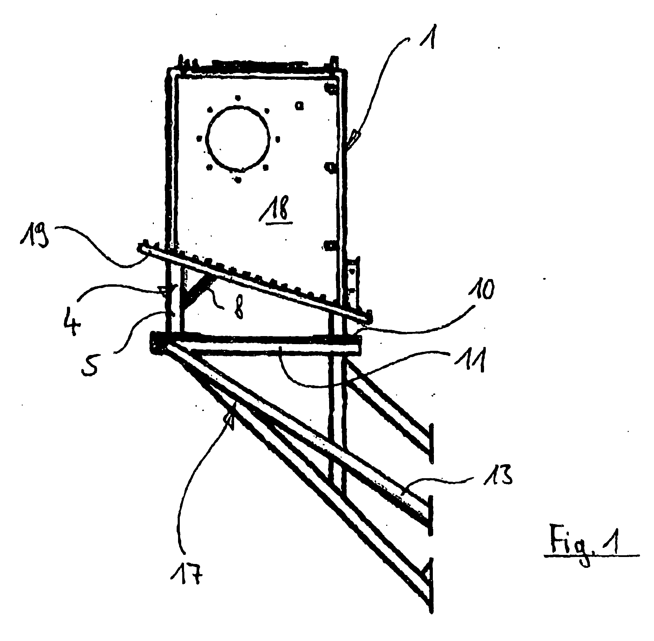

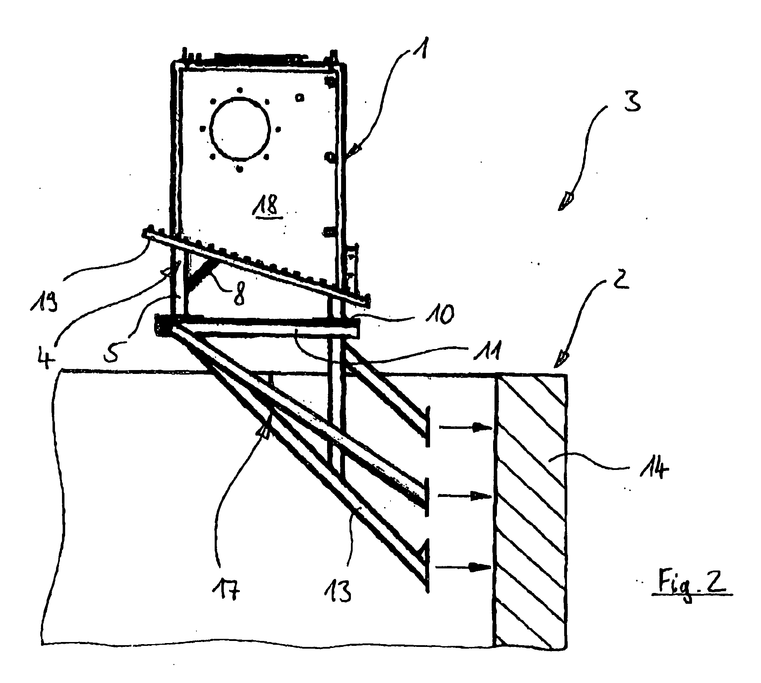

In den Fig. 1 bis 4 ist schematisch die Montage eines erfindungsgemäßen Biogasanlagen-Serviceschachtes 1 in einem Fermenterbehälter 2 einer Biogasanlage 3 dargestellt.

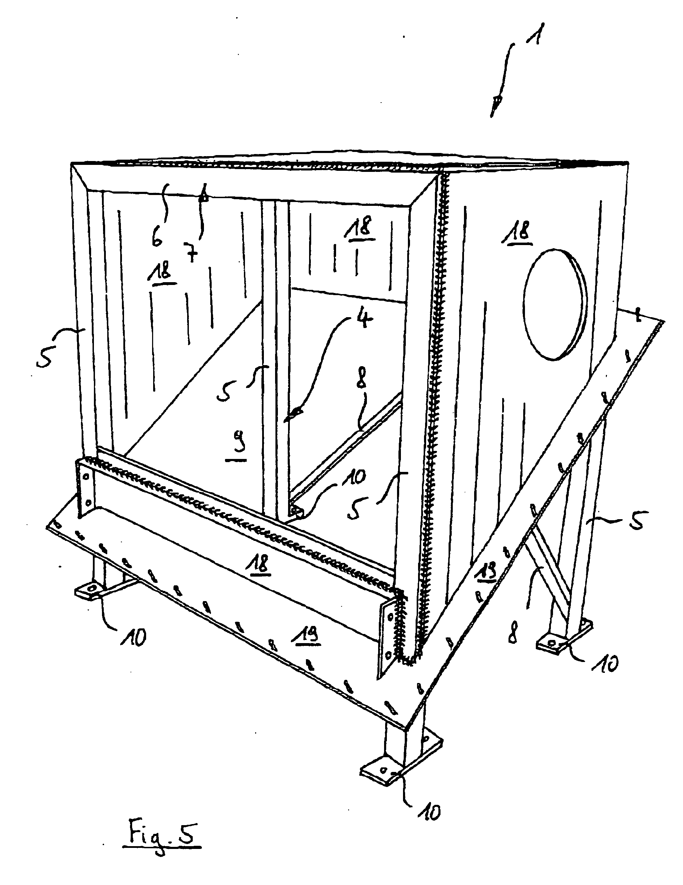

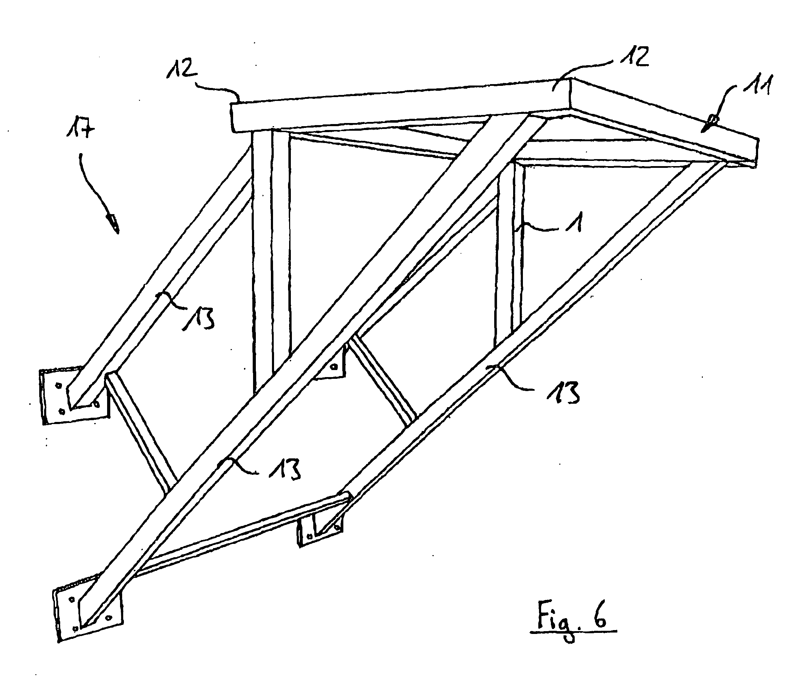

Wie dies den Fig. 1 bis 4 in Verbindung mit den Fig. 5 und 6 entnommen wer den kann, weist der Serviceschacht 1 einen rechteckförmigen Grundriss mit einer gestellartigen Tragstruktur 4 auf, die in den vier Eckbereichen jeweils durch Hohlproflträger ausgebildete Vertikalträger 5 aufweist, die an ihrem oberen Tragstrukturende mittels hier nicht im Detail dargestellter horizontal verlaufender Querträger 6 zu einem die Tragstruktur 4 insgesamt versteifenden Tragstruktur-Rahmenbereich 7 ausgebildet sind. Die Vertikalträger 5 können, wie dies insbesondere auch der Fig. 5 entnommen werden kann, jeweils noch mittels Versteifungsstreben 8 versteift sein, die jedoch nur zwischen unmittelbar benachbarten Vertikalträgern 5 verlaufen, um den Serviceschacht-Innenraum 9 freizuhalten.

Die Vertikalträger 5 weisen an ihren unteren Enden horizontale Flanschplatten 10 auf, mittels denen der Serviceschacht 1, wie dies insbesondere aus den Fig. 1 bis 4 ersichtlich ist an einem eine horizontale Anschlussebene ausbildenden horizontalen Tragkonsolenrahmen 11 einer Tragkonsole 17 mittels einer Schraubverbindung, die hier nicht dargestellt ist, festgelegt wird. Dieser horizontale Tragkonsolenrahmen 11 weist einen rechteckförmigen Grundriss auf, der im wesentlichen von seinen Abmessungen her identisch mit demjenigen der Tragstruktur 4 ist, so dass sich die horizontalen Flanschplatten 10 jeweils in Eckbereichen des horizontalen Tragkonsolenrahmens 11 abstützen.

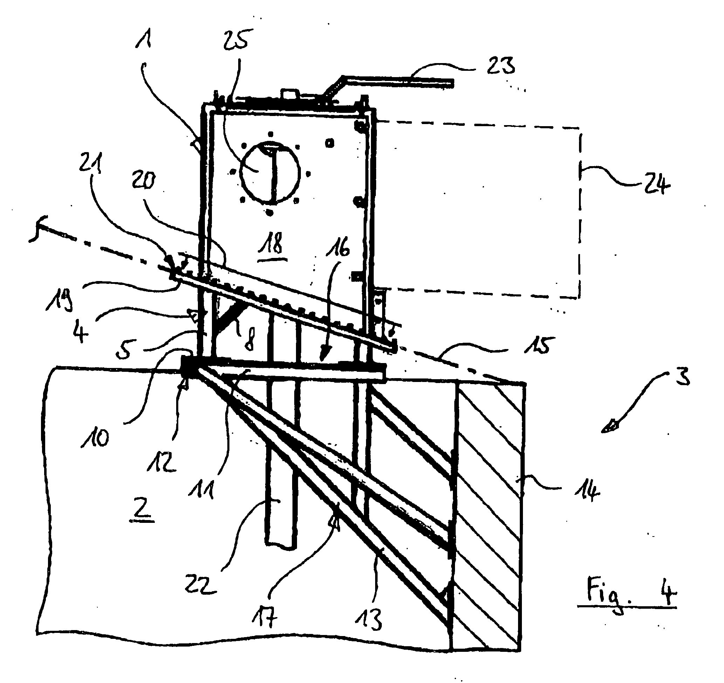

Von diesem horizontalen Tragkonsolenrahmen 11 gehen jeweils von den Eckbereichen 12 Diagonalstreben 13 aus, die jeweils so schräg bzw. diagonal verlaufend zu einer Fermenterbehälter-Seitenwand 14 geführt sind, dass eine in einem gegen die Horizontale geneigten, schräg verlaufenden Foliendach 15 ausgebildete Serviceöffnung 16 freigehalten wird. Nähere Einzelheiten zum Foliendach 15 werden nachfolgend in Verbindung mit der Fig. 4 noch näher erläutert.

Die Serviceschacht-Tragstruktur 4 des Serviceschachtes 1 wird in denjenigen Bereichen, in denen diese im montierten Zustand oberhalb bzw. außerhalb des Foliendaches 15 liegt mit Edelstahlblechen als Außenhaut 18 beplankt bzw. verschweißt, wobei diese Außenhaut am unteren Außenhautrandbereich so umgebördelt ist (Fig. 6), dass diese eine erste Klemmleiste 19 integral ausbildet, zwischen der und einer hier nicht im Detail gezeigten zweiten Klemmleiste 20 ein die Serviceöffnung 16 im Foliendach 15 begrenzender Folienrandbereich 21 gasdicht verklemmt werden kann (Fig. 4).

In diesem in der Fig. 4 dargestellten fertig montierten Zustand des Zusammenbaus aus Serviceschacht 1 und Tragkonsole 17 ist somit lediglich der oberhalb des Foliendaches 15 liegende Bereich des Serviceschachtes 1 mit einer Außenhaut versehen und somit lediglich im Bereich der beiden Klemmleisten 19, 20 eine gasdichte Anbindung des Foliendaches 15 an den Serviceschacht 1 erforderlich.

Das Foliendach 15 selbst ist hier an einem oberen Fermenterbehälter-Seitenwandbereich 14 festgelegt und zeltdachartig zu einer fermenterbehältermittig angeordneten Dachspitze hin gegen die Horizontale geneigt schräg nach oben verlaufend ausgebildet.

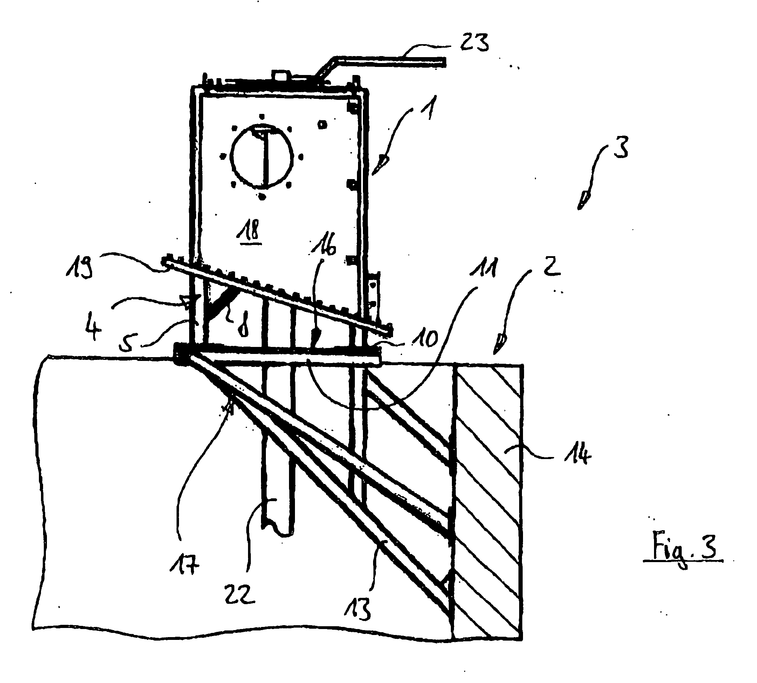

Sobald der Zusammenbau aus Serviceschacht 1 und Tragkonsole 17 montiert ist (Fig. 3) kann eine Stützstange 22 montiert werden, die gasdicht in einem Serviceschachtdachbereich gelagert und abgestützt und von außen mit einer Betätigungseinrichtung 23 z. B. manuell betätigbar ist, um die Stützstange um ihre Längsachse zu verdrehen, und zwar innerhalb vorgegebener Einstellbereiche. An dieser Stützstange 22 ist eine Rühreinheit, die hier nicht dargestellt ist, höhenverstellbar gehalten und kann durch die Serviceöffnung 16 hindurch in den Serviceschacht-Innenraum 9 bewegt werden, z. B. mittels einer hier ebenfalls nicht dargestellten und an der Stützstange 22 serviceschachtseitig angeordneten Seilzuganordnung, um durch die verschließbare Zugangstür 24 Wartungs- und/oder Reparaturarbeiten an der Rühreinheit vornehmen zu können. Dazu kann die Serviceöffnung 16 in der eingangs in Verbindung mit dem gattungsbildenden Stand der Technik geschilderten Art und Weise im Wesentlichen gasdicht abgedeckt werden, was hier aber nicht im Detail dargestellt ist.

Wie dies der Fig. 6 entnommen werden kann, ist die Stützstange in einem außermittigen Randbereich durch die Serviceöffnung 16 hindurchgeführt und ist mit dem oberen Stützstangenende an einem Wandbereich im Inneren des Serviceschachtes 1 verdrehbar gehalten und abgestützt. Letzteres ist hier nicht im Detail dargestellt.

In der die Außenhaut ausbildenden Blechbeplankung des Serviceschachtes 1 können ferner noch Sichtfenster 25 angeordnet sein, um von der Fermenterbehälteraußenseite her einen Einblick in den Fermenterbehälferinnenraum zu haben, ohne das man hierfür die Zugangstür 24 öffnen muss.

Wie dies der Fig. 5 weiter entnommen werden kann, ist die hier aus Übersichtlichkeitsgründen nicht dargestellte Zugangstür 24 in dem die größte Fläche aufweisenden und zum Fermenterbehälterrand hinweisenden Serviceschachtbereich ausgebildet, um eine möglichst große Zugangsöffnung zu schaffen.

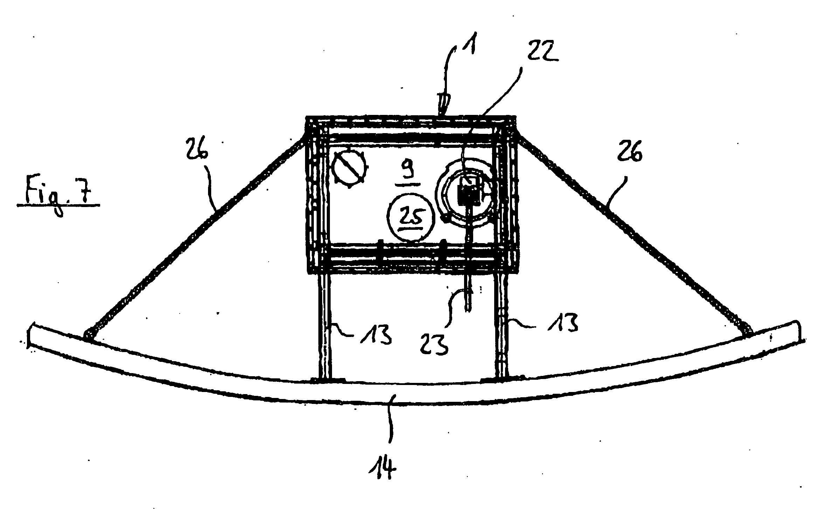

Eine besonders vorteilhafte Versteifung der gesamten Konstruktion ergibt sich auch durch das Vorsehen von zusätzlichen, den Zusammenbau aus Serviceschacht 1 und Tragkonsole 17 versteifende Versteifungsstreben 26, die einerseits an der Tragkonsole 17 und andererseits an der Fermenterbehälter-Seitenwand 14 angebunden sind (Fig. 7).

## Patentansprüche

1. Biogasanlagen-Serviceschacht für einen Fermenterbehälter einer Biogasanlage,
wobei der Serviceschacht (1) als gasdichte Abdeckung domartig über einer in einer Fermenterbehälterabdeckung ausgebildeten Serviceöffnung (16) angebracht ist, durch die hindurch eine Rühreinheit, die an einer im Fermenterbehälter vertikal gehaltenen und mit einem oberen Stützstangenende in einen Serviceschacht-Innenraum (9) geführte Stützstange (22) höhenverstellbar geführt und gehalten ist, aus dem Fermenterbehälter heraus in den durch wenigstens eine gasdicht verschließbare Zugangsöffnung zugänglichen Serviceschacht-Innenraum (9) bewegbar ist,
**dadurch gekennzeichnet,**
**dass** die Fermenterbehälterabdeckung durch ein gegen die Horizontale geneigtes Fermenterbehälter-Foliendach (15) gebildet ist, durch das der Serviceschacht (1) mit einem unteren Schachtende unter Ausbildung der Serviceöffnung (16) gasdicht hindurch und in den Fermenterbehälterinnenraum geführt und sowie dort festgelegt ist.

2. Biogasanlagen-Serviceschacht nach Anspruch 1, **dadurch gekennzeichnet, dass** das untere Schachtende an einer eine horizontale Anschlussebene aufweisenden und im Fermenterbehälterinnenraum angeordneten Tragkonsole (17) abgestützt und festgelegt ist.

3. Biogasanlagen-Serviceschacht nach Anspruch 1 oder Anspruch 2, **dadurch gekennzeichnet, dass** das Foliendach (15) mit seinem die Serviceöffnung (16) umgebenden Folienrandbereich (21) gasdicht zwischen mehreren dem Serviceschacht (1) zugeordneten und um diesen umlaufenden Klemmleisten (19,20) verklemmt ist.

4. Biogasanlagen-Serviceschacht nach einem der Ansprüche 1 bis 3, **dadurch gekennzeichnet, dass** der Serviceschacht (1) eine rahmen- oder gestellartige Serviceschacht-Tragstruktur (4) aufweist, die wenigstens in dem das Foliendach (15) nach außen überragenden Bereich mit einer Außenhaut (18), insbesondere aus einem Edelstahlblech beplankt bzw. ummantelt ist.

5. Biogasanlagen-Serviceschacht nach Anspruch 3 und 4, **dadurch gekennzeichnet, dass** eine im montierten Zustand des Serviceschachtes (1) untere Klemmleiste (19) der beiden Klemmleisten (19, 20) integraler Bestandteil der Außenhaut (18) ist, insbesondere deren dachseitigen Außenhaut-Endbereich ausbildet, den die Serviceschacht-Tragstruktur (4) nach unten hin in Abhängigkeit von der durch die Foliendachneigung vorgegebenen Neigung der unteren Klemmleiste (19) gegen die Horizontale mit einem unteren Tragstrukturende überragt.

6. Biogasanlagen-Serviceschacht nach Anspruch 4 oder Anspruch 5, **dadurch gekennzeichnet,**
**dass** der Serviceschacht (1) einen rechteckförmigen Grundriss mit einem in jedem Eckbereich angeordneten Vertikalträger (5) als Bestandteil der Serviceschacht-Tragstruktur (4) aufweist,
**dass** die Vertikalträger (5) vorzugsweise am im montierten Zustand des Serviceschachtes (1) oberen Endbereich der Serviceschacht-Tragstruktur (4) miteinander durch horizontale Querträger (6) zu einem rechteckförmigen Tragstruktur-Rahmenbereich (7) verbunden sind,
**dass** die Vertikalträger (5) am im montierten Zustand des Serviceschachtes (1) unteren Endbereich der Serviceschacht-Tragstruktur (4) horizontale Flanschplatten (10) zur Festlegung an der die horizontale Anschlussebene aufweisenden Tragkonsole (17) aufweisen.

7. Biogasanlagen-Serviceschacht nach Anspruch 6, **dadurch gekennzeichnet, dass** zwischen den jeweils benachbarten Vertikalträgern (5) wenigstens eine Versteifungsstrebe (8) vorgesehen ist.

8. Biogasanlagen-Serviceschacht nach Anspruch 6 oder Anspruch 7, **dadurch gekennzeichnet, dass** die Tragkonsole (17) zur Ausbildung der horizontalen Anschlussebene einen dem Serviceschacht (1) unmittelbar zugeordneten und im montierten Zustand einen rechteckförmigen Grundriss aufweisenden, horizontalen, insbesondere durch Querträger gebildeten Konsolenrahmen (11) aufweist, auf dem die Serviceschacht-Tragstruktur (4) mittels der an den Vertikalträgern (5) angeordneten horizontalen Flanschplatten (10) festlegbar, insbesondere verschraubbar ist.

9. Biogasanlagen-Serviceschacht nach Anspruch 8, **dadurch gekennzeichnet, dass** die Tragkonsole vier von den Eckbereichen (12) des im montierten Zustand in einer Horizontalebene liegenden Konsolenrahmens (11) ausgehende Diagonalstreben (13) aufweist, die unter Freigabe der Serviceöffnung (16) vom Fermenterbehälter-Innenraum ausgehend, vorzugsweise jeweils parallel zueinander verlaufen und mit ihren freien Enden an einer Seitenwand (14) des Fermenterbehälters (2) festgelegt sind.

10. Biogasanlagen-Serviceschacht nach einem der Ansprüche 1 bis 9, **dadurch gekennzeichnet, dass** die Tragkonsole (17) im montierten Zustand zusätzlich auf einander in Fermenterbehätterumfangsrichtung gesehen gegenüberliegenden Konsolenseiten mit in der Draufsicht schräg verlaufenden Versteifungsstreben (26) versteift ist, die einerseits an der Tragkonsole (17) und andererseits an der Fermenterbehälter-Seitenwand (14) angebunden sind.

11. Biogasanlagen-Serviceschacht nach einem der Ansprüche 1 bis 10, **dadurch gekennzeichnet, dass** die gasdicht verschließbare Serviceschacht-Zugangsöffnung (24), vorzugsweise eine Zugangsklappe oder -tür, in dem aufgrund der Foliendachneigung die größte Fläche aufweisenden und zum Fermenterbehälterrand gerichteten Serviceschachtbereich ausgebildet ist.

12. Biogasanlagen-Serviceschacht nach einem der Ansprüche 1 bis 11, **dadurch gekennzeichnet, dass** die Stützstange (22) in einem mittigen oder außermittigen Randbereich durch die Serviceöffnung (16) hindurchgeführt und mit dem oberen Stützstangenende an einem Wandbereich des Serviceschachtes (1) verdrehbar gehalten und abgestützt ist.

13. Biogasanlagen-Serviceschacht nach Anspruch 12, **dadurch gekennzeichnet, dass** die Stützstange (22) von außerhalb des Serviceschachtes (1) mittels einer gasdicht durch die Serviceschachtwand geführten und mittels dem oberen Stützstangenende gekoppelten bzw. Bestandteil desselben bildenden Verdreheinrichtung (23) verdrehbar ist.

14. Biogasanlagen-Serviceschacht nach Anspruch 12 oder Anspruch 13, **dadurch gekennzeichnet, dass** zur Höhenverstellung der einen Antrieb und einen Rührer aufweisenden Rühreinheit eine Verstelleinrichtung, insbesondere eine mittels einer Kurbel betätigbare Seilzuganordnung, vorgesehen ist, die an der Stützstange (22) angeordnet und im Serviceschacht-Innenraum aufgenommen ist.

## Claims

1. Biogas plant service shaft for a fermenter tank of a biogas plant,
with the service shaft (1) being attached as a gas-tight cover in the manner of a dome over a service opening (16) which is formed in a fermenter tank cover and through which a stirring unit, which is guided and held in a height-adjustable manner on a support rod (22) which is held vertically in the fermenter tank and is guided with an upper support rod end in a service shaft interior space (9), can be moved out of the fermenter tank into the service shaft interior space (9) which is accessible through at least one access opening which can be closed off in a gas-tight manner,
**characterized**
**in that** the fermenter tank cover is formed by a fermenter tank film roof (15) which is inclined relative to the horizontal and through which the service shaft (1) is guided in a gas-tight manner with a lower shaft end so as to form the service opening (16) and is guided into and fixed in the fermenter tank interior space.

2. Biogas plant service shaft according to Claim 1, **characterized in that** the lower shaft end is supported and fixed on a support bracket (17) which has a horizontal connection plane and is arranged in the fermenter tank interior space.

3. Biogas plant service shaft according to Claim 1 or Claim 2, **characterized in that** the film roof (15) is clamped, with its film edge region (21) which surrounds the service opening (16), in a gas-tight fashion between a plurality of clamping strips (19, 20) which are assigned to and run around the service shaft (1).

4. Biogas plant service shaft according to one of Claims 1 to 3, **characterized in that** the service shaft (1) has a frame-like or framework-like service shaft support structure (4) which, at least in the region which projects outward beyond the film roof (15), is covered or encased with an outer skin (18) made in particular from a high-grade steel sheet.

5. Biogas plant service shaft according to Claim 3 and 4, **characterized in that** a lower clamping strip (19) of the two clamping strips (19, 20) in the assembled state of the service shaft (1) is an integral constituent part of the outer skin (18) and in particular forms the roof-side outer skin end region of said outer skin (18), with the service shaft support structure (4) projecting, with a lower support structure end, downward beyond said roof-side outer skin end region as a function of the inclination, which is defined by the film roof inclination, of the lower clamping strip (19) relative to the horizontal.

6. Biogas plant service shaft according to Claim 4 or Claim 5, **characterized**
**in that** the service shaft (1) has a rectangular outline with a vertical support (5), arranged in each corner region, as a constituent part of the service shaft support structure (4),
**in that** the vertical supports (5) are preferably connected to one another at the upper end region of the service shaft support structure (4) in the assembled state of the service shaft (1) by means of horizontal transverse members (6) to form a rectangular support structure frame region (7),
**in that** the vertical supports (5) have, at the lower end region of the service shaft support structure (4) in the assembled state of the service shaft (1), horizontal flange plates (10) for fixing to the support bracket (17) which has the horizontal connection plane.

7. Biogas plant service shaft according to Claim 6, **characterized in that** at least one stiffening strut (8) is provided between the in each case adjacent vertical supports (5).

8. Biogas plant service shaft according to Claim 6 or Claim 7, **characterized in that**, in order to form the horizontal connection plane, the support bracket (17) has a bracket frame (11) which is directly assigned to the service shaft (1) and, in the assembled state, has a rectangular outline, is horizontal and is in particular formed by transverse members, to which bracket frame (11) the service shaft support structure (4) can be fixed, in particular screwed, by means of the horizontal flange plates (10) which are arranged on the vertical supports (5).

9. Biogas plant service shaft according to Claim 8, **characterized in that** the support bracket has four diagonal struts (13) which proceed from the corner regions (12) of the bracket frame (11) which, in the assembled state, lies in a horizontal plane, which diagonal struts (13), while leaving the service opening (16) free, proceed from the fermenter tank interior space, preferably run in each case parallel to one another and are fixed with their free ends to a side wall (14) of the fermenter tank (2).

10. Biogas plant service shaft according to one of Claims 1 to 9, **characterized in that** the support bracket (17), in the assembled state, is additionally stiffened on opposite bracket sides as viewed in the fermenter tank peripheral direction with stiffening struts (26) which run obliquely in plan view and which are attached at one side to the support bracket (17) and at the other side to the fermenter tank side wall (14).

11. Biogas plant service shaft according to one of Claims 1 to 10, **characterized in that** the service shaft access opening (24), preferably an access flap or door, which can be closed off in a gas-tight fashion, is formed **in that** service shaft region which, on account of the film roof inclination, has the largest area and is aligned towards the fermenter tank edge.

12. Biogas plant service shaft according to one of Claims 1 to 11, **characterized in that** the support rod (22), in a central or non-central edge region, is guided through the service opening (16) and, with the upper support rod end, is rotatably held and supported on a wall region of the service shaft (1).

13. Biogas plant service shaft according to Claim 12, **characterized in that** the support rod (22) can be rotated from outside the service shaft (1) by means of a rotary device (23) which is guided through the service shaft wall in a gas-tight fashion and is coupled by means of the upper support rod end or forms a constituent part thereof.

14. Biogas plant service shaft according to Claim 12 or Claim 13, **characterized in that** an adjustment device, in particular a Bowden cable arrangement which can be actuated by means of a crank, is provided for the height adjustment of the stirring unit which has a drive and a stirrer, which adjustment device is arranged on the support rod (22) and is held in the service shaft interior space.

## Revendications

1. Elément de regard d'une installation de biogaz pour le réservoir à fermentateur d'une installation de biogaz,
l'élément de regard (1) étant monté sous forme de couvercle étanche au gaz comme un dôme sur un orifice de regard (16) réalisé dans un couvercle du réservoir de fermentateur, orifice à travers lequel une unité de brassage guidée de manière à être réglable en hauteur, maintenue verticalement dans le fermentateur et guidée avec une extrémité de barre d'appui (22) supérieure dans un espace intérieur (9) d'élément de regard, peut se déplacer du réservoir du fermentateur dans l'espace intérieur de l'élément de regard (9), accessible par au moins un orifice d'accès pouvant être fermé de manière à être étanche au gaz
**caractérisé en ce que**
le couvercle du fermentateur est formé par un toit en film (15) de réservoir de fermentateur incliné par rapport à l'horizontale, à travers lequel l'élément de regard (1) est guidé avec une extrémité inférieure de l'élément de regard en assurant l'étanchéité au gaz et en réalisant l'orifice de regard (16), puis guidé dans l'espace intérieur du réservoir de fermentateur, où il est fixé.

2. Elément de regard d'une installation de biogaz selon la revendication 1, **caractérisé en ce que** l'extrémité inférieure de l'élément de regard est appuyée et fixée sur une console de support (17) comportant un plan de raccord horizontal et disposée dans l'espace intérieur du réservoir de fermentateur.

3. Elément de regard d'une installation de biogaz selon la revendication 1 ou la revendication 2, **caractérisé en ce que** le toit en film (15) est bloqué avec sa zone de bordure de film (21) qui entoure l'orifice de regard (16) entre plusieurs barres de serrage (19, 20) affectées à l'élément de regard (1) et placées sur son pourtour, de manière à être étanche au gaz.

4. Elément de regard d'une installation de biogaz selon l'une quelconque des revendications 1 à 3, **caractérisé en ce que** l'élément de regard (1) comporte une structure de support ressemblant à un cadre ou à un châssis (4) revêtue et/ou enveloppée, au moins dans la zone qui dépasse du toit en film (15) vers l'extérieur, d'un revêtement (18), en particulier d'une tôle en acier inoxydable.

5. Elément de regard d'une installation de biogaz selon les revendications 3 et 4, **caractérisé en ce que**, à l'état monté de l'élément de regard (1), une barre de serrage inférieure (19) des deux barres de serrage (19, 20) est partie intégrante du revêtement (18), formant en particulier sa zone d'extrémité du revêtement côté toit, dont la structure de support de l'élément de regard (4) dépasse vers le bas avec une extrémité inférieure de structure de support, en fonction de l'inclinaison de la barre de serrage inférieure (19) par rapport à l'horizontale déterminée par l'inclinaison du toit en film.

6. Elément de regard d'une installation de biogaz selon la revendication 4 ou la revendication 5, **caractérisé en ce que**
l'élément de regard (1) comporte un plan en forme de rectangle avec un support vertical (5) disposé dans chaque zone de coin comme composante de la structure de support de l'élément de regard (4),
les supports verticaux (5) sont de préférence reliés les uns aux autres par des supports transversaux (6) horizontaux sur la zone d'extrémité supérieure de la structure de support de l'élément de regard à l'état monté de l'élément de regard (1), pour former une zone de cadre de structure de support (7) en forme ce rectangle,
les supports verticaux (5) comportent sur la zone d'extrémité inférieure de la structure de support (4) de l'élément de regard à l'état monté de l'élément de regard (1) des plaques à brides (10) pour la fixation sur la console de support (17) qui comporte le plan de raccord horizontal.

7. Elément de regard d'une installation de biogaz selon la revendication 6, **caractérisé en ce qu'**au moins une contrefiche de renfort (8) est prévue entre les supports verticaux (5) respectivement voisins.

8. Elément de regard d'une installation de biogaz selon la revendication 6 ou la revendication 7, **caractérisé en ce que** la console de support (17) pour la réalisation du plan de raccord horizontal comporte un cadre de console (11) horizontal, formé en particulier par des supports transversaux, directement affecté à l'élément de regard (1) et comportant à l'état monté un plan en forme de rectangle, sur lequel la structure de support (4) de l'élément de regard peut être fixée, en particulier vissée, au moyen des plaques à brides (10) horizontales disposées sur les supports verticaux (5).

9. Elément de regard d'une installation de biogaz selon la revendication 8, **caractérisé en ce que** la console de support comporte quatre contrefiches diagonales (13) partant des zones de coin (12) du cadre de console (11) situé dans un plan horizontal à l'état monté, qui, en dégageant l'orifice de regard (16) à partir de l'espace intérieur du réservoir de fermentateur, sont respectivement de préférence parallèles et fixées avec leurs extrémités libres à une paroi latérale (14) du réservoir de fermentateur (2).

10. Elément de regard d'une installation de biogaz selon l'une quelconque des revendications 1 à 9, **caractérisé en ce que** la console de support (17) est également renforcée à l'état monté, sur des côtés de la console qui se font face vues dans le sens du pourtour du réservoir de fermentateur, par des contrefiches de renfort (26) obliques vues de dessus qui sont reliées d'une part à la console de support (17) et, d'autre part, à la paroi latérale (14) du réservoir de fermentateur.

11. Elément de regard d'une installation de biogaz selon l'une quelconque des revendications 1 à 10, **caractérisé en ce que** l'orifice d'accès de l'élément de regard (24) pouvant être fermé de manière à être étanche au gaz, de préférence un volet ou une porte d'accès, est réalisé dans la zone de l'élément de regard, qui comporte la plus grande surface en raison de l'inclinaison du toit en film et est dirigée vers le bord du réservoir de fermentateur.

12. Elément de regard d'une installation de biogaz selon l'une quelconque des revendications 1 à 11, **caractérisé en ce que** la barre d'appui (22) est guidée à travers l'orifice de regard (16) dans une zone de bordure centrée ou excentrée et maintenue de manière à permettre la torsion et appuyée sur une zone de paroi (1) avec l'extrémité supérieure de la barre d'appui.

13. Elément de regard d'une installation de biogaz selon la revendication 12, **caractérisé en ce que** la barre d'appui (22) peut subir une torsion grâce à un dispositif de torsion (23) guidé à travers la paroi de l'élément de regard de manière à être étanche au gaz en dehors de l'élément de regard (1) et couplé au moyen de l'extrémité supérieure de la barre d'appui et/ou formant un élément de cette dernière.

14. Elément de regard d'une installation de biogaz selon la revendication 12 ou la revendication 13, **caractérisé en ce que**, pour régler la hauteur de l'unité de brassage comportant un entraînement et un mélangeur, un dispositif de réglage, en particulier un dispositif à câble Bowden pouvant être actionné au moyen d'une manivelle, qui est disposé sur la barre d'appui (22) et logé dans l'espace intérieur de l'élément de regard, est prévu.
